(19) Europäisches Patentamt
European Patent Office
Office européen des brevets

(11) EP 0 557 199 B1

(12) # FASCICULE DE BREVET EUROPEEN

(45) Date de publication et mention de la délivrance du brevet: **23.01.2002 Bulletin 2002/04**

(51) Int Cl.[7]: **C07K 17/10**, A61K 38/55, A61K 47/30

(21) Numéro de dépôt: **93400421.9**

(22) Date de dépôt: **18.02.1993**

(54) **Conjugés polyéthylèneglycol-hirudine, leur procédé de préparation et leur emploi pour le traitement des thromboses**

Polyethylenglycol-Hirudin-Konjugate, deren Verfahren zur Herstellung und Verwendung für die Heilung von Thrombosen

Polyethyleneglycol hirudin conjugate, process of preparation and use in the cure of thrombosis

(84) Etats contractants désignés:
**AT BE CH DE DK ES FR GB IT LI MC NL PT SE**

(30) Priorité: **20.02.1992 FR 9201938**

(43) Date de publication de la demande:
**25.08.1993 Bulletin 1993/34**

(73) Titulaire: **HOECHST AKTIENGESELLSCHAFT
65926 Frankfurt am Main (DE)**

(72) Inventeur: **Bischoff, Rainer
F-67400 Illkirch-Graffenstaden (FR)**

(74) Mandataire: **Warcoin, Jacques et al
Cabinet Régimbeau 20, rue de Chazelles
75847 Paris cedex 17 (FR)**

(56) Documents cités:
**EP-A- 0 304 311**
**WO-A-89/01033**
**WO-A-91/08229**

- **V. R. Meyer, Praxis der Hochleistungschromatographie, Verlag Salle und Sauerländer, 7. Auflage, Seiten 110, 111**
- **A. Abuchowski et al, Cancer Biochem. Biophys., 1984, vol. 7, pp 175-186, "Cancer Therapy with Chemically Modified enzymes. I. Antitumor Properties of Polyethylene Glycol-Asparaginase Conjugates"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 557 199 B1

## Description

La présente invention concerne un procédé de préparation d'un conjugué polyéthylèneglycol(ou dérivés)-peptide, ci-après PEG-peptide, et en particulier d'un conjugué polyéthylèneglycol(ou dérivés)-hirudine (PEG-hirudine).

Les composés tels que le polyéthylèneglycol et ses dérivés, ci-après PEG, ou le Dextran sont connus pour ne pas être immunogènes chez l'homme et leur utilisation en association avec des protéines à usage pharmacologique a déjà été proposé pour prolonger la demi-vie de ces protéines in vivo.

Ainsi, un peptide tel que l'hirudine dont la source principale se trouve dans les glandes salivaires des sangsues médicinales sous forme d'un mélange de peptides de 65 et 66 acides aminés, connue pour ses propriétés inhibitrices très spécifiques et très efficaces de la thrombine, est éliminé très rapidement de la circulation sanguine. Son temps de demi-vie est d'environ 1h. Il est donc très intéressant de prolonger la demi-vie in vivo de l'hirudine en particulier pour son utilisation dans la prophylaxie des thromboses.

Pour augmenter la demi-vie de l'hirudine in vivo, un conjugué Dextran-hirudine a été produit par P. Crause **(EP-A-0 345 616)**. Bien que la demi-vie du Dextran-hirudine soit augmentée in vivo chez le rat par rapport à la demi-vie de l'hirudine libre, le rendement du procédé de couplage de l'hirudine au Dextran est très faible puisqu'on observe une perte de plus 70% de l'activité de l'hirudine conjuguée.

Le couplage PEG-peptide est également connu et est généralement obtenu par une liaison covalente.

WO-A-91 08229 déscrit un procédé de préparation d'un conjugé d'un polyethylene glycol- dérivé avec un dérivé de hirudine comprenant les étapes d'activation du PEG, de récuperation du PEG activé et le couplage du PEG activé avec un peptide.

Le procédé de couplage du PEG à un peptide comprend les deux étapes principales d'activation du PEG, et de couplage du PEG activé au peptide.

La première étape d'activation est généralement limitante pour le rendement final en conjugué obtenu.

En effet, la technique antérieure propose des procédés d'activation du PEG qui nécessitent une étape de purification et de récupération du PEG activé, réalisée avec des rendements faibles.

Ainsi Beauchamp et al. (Analytical Biochemistry 1983, 131, 25-33) décrivent un procédé d'activation du PEG par réaction avec du carbonyldiimidazole dans le dioxane, qui comprend une étape de purification du PEG activé par dialyse extensive contre l'eau et lyophilisation.

Cette dialyse étant extensive pose des problèmes pour son application à l'échelle industrielle. Par ailleurs, le PEG activé se désactive en milieu aqueux, ce qui entraine une baisse du rendement de la réaction d'activation due à l'étape de dialyse.

La présente invention concerne donc un procédé de préparation d'un conjugué PEG-peptide d'utilisation simple et rapide, applicable à l'échelle industrielle. La présente invention concerne avantageusement un procédé de préparation d'un conjugué PEG-hirudine, dont au moins 80% de l'hirudine est couplé au PEG et les conjugués obtenus ont une activité voisine de 100% par rapport à l'hirudine de départ.

Le procédé selon la présente invention comprend les étapes connues d'activation du PEG dans un solvant anhydre approprié, de récupération du PEG et sa réaction avec un peptide.

Par PEG, ou polyéthylèneglycol et ses dérivés, on entend principalement le polyéthylèneglycol et les monoéthers aliphatiques inférieurs en $C_1$-$C_6$ du polyéthylèneglycol, également les monoéthers aromatiques ou monoesters du polyéthylèneglycol, ainsi que les dérivés polyglycolés comprenant au moins 90% de motifs éthylèneglycol par rapport au nombre total de motifs monomères.

Il a été trouvé d'une manière inattendue que le PEG activé à l'aide de l'agent d'activation carbonyldiimidazole pouvait être simplement purifié par précipitation à partir du mélange d'activation à l'aide d'un solvant organique hydrophobe et rester actif pour l'étape ultérieure de condensation sur un peptide.

Le solvant organique hydrophobe est choisi parmi les solvants aprotiques apolaires, parmi lesquels on trouve les éthers aliphatiques et les hydrocarbures.

D'une manière avantageuse, ce solvant organique hydrophobe est sélectionné parmi l'éther éthylique, le pentane, l'hexane ou l'heptane, de préférence l'éther éthylique.

Le PEG activé est alors récupéré par filtration puis lyophilisé.

La réaction d'activation du PEG est effectuée dans un solvant anhydre approprié, préférentiellement dans un solvant aprotique polaire.

Ce solvant anhydre approprié est généralement choisi parmi le dioxane, le diméthylformamide, l'acétonitrile, le diméthylsulfoxyde ou le tétrahydrofurane.

D'une manière préférentielle, le PEG est activé à l'aide de carbonyldiimidazole comme agent d'activation.

Le rapport molaire entre l'agent d'activation et le PEG, lors de l'activation de ce dernier est compris entre 5 et 50, de préférence entre 10 et 20.

Le PEG employé dans le procédé selon la présente invention a un poids moléculaire compris entre 2.000 et 100.000, avantageusement compris entre 2.000 et 50.000, et est choisi parmi le polyéthylèneglycol et les monoesters

aliphatiques en $C_1$-$C_6$ du polyéthylèneglycol.

Le PEG activé obtenu par réaction avec le carboxydiimide peut alors être représenté par la formule générale I, suivante :

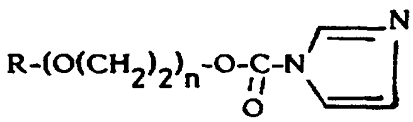

(I)

R pouvant être notamment, d'une manière indifférente, un atome d'hydrogène, un radical alkyle en $C_1$-$C_6$ ou un groupement carboxyimidazole, n étant compris entre environ 45 et environ 2.400.

Le couplage avec le peptide est effectué à pH légèrement alcalin en substituant le noyau imidazole par une fonction amine libre du peptide.

Le conjugué obtenu peut alors être représenté par la formule générale II, suivante :

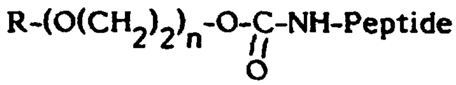

(II)

R et n étant définis précédemment.

D'une manière particulièrement avantageuse, le procédé selon la présente invention est employé pour la préparation d'un conjugué PEG-hirudine.

Par hirudine, on entend l'hirudine naturelle extraite des glandes salivaires des sangsues médicinales, ses variants naturels dont certains ont été désignés par HV1, HV2 et HV3, ainsi que les variants et analogues produits par génie génétique et conservant leur activité anti-thrombotique.

La préparation de ces peptides par génie génétique a été décrite en particulier dans les demandes de brevets EP-A-0 158 564, EP-A-0 200 655, EP-A-0 273 800 et EP-A-0 332 529.

L'hirudine comprend plusieurs groupements Lysine dans sa séquence peptidique. Le PEG activé peut donc se fixer de façon aléatoire sur la fonction N-terminale du peptide ou l'une quelconque des fonctions amine des différents groupements Lysine.

Or, de manière inattendue, le PEG activé ne se fixe pas sur l'amine N-terminale de l'hirudine, ce qui permet d'obtenir un conjugué avec une activité anti-thrombotique voisine de celle du peptide seul.

En conséquence, la présente invention concerne également un procédé de préparation d'un conjugué PEG-hirudine qui comprend :

a) l'activation du PEG par réaction avec le carbonyldiimidazole dans un solvant anhydre approprié,
b) la récupération du PEG activé par précipitation à l'aide d'un solvant hydrophobe,
c) la condensation du PEG activé sur l'hirudine ou ses variants, en milieu légérement alcalin, et
d) la purification du conjugué obtenu.

Les étapes a), b) et c) de préparation du PEG activé et de sa condensation sur le peptide sont effectuées dans les conditions opératoires décrites précédemment.

L'étape d) de purification du conjugué PEG-hirudine obtenu est généralement effectuée par chromatographie en deux étapes :

d1) par chromatographie sur échange d'anions pour éliminer l'excès de PEG qui n'aurait pas réagit, suivie d'une
d2) chromatographie de phase inverse pour séparer le conjugué obtenu de l'hirudine libre résiduelle.

La résine échangeuse d'anions employée pour d1) est avantageusement une D-sépharose ou Q-sépharose, et la phase inverse de d2) une phase RP300 ou RP8.

Le rendement en hirudine conjuguée obtenue,présentant une activité anti-thrombotique,est supérieur à 60%.

D'une manière avantageuse, le procédé selon la présente invention est employé sur le variant de l'hirudine HV2-Lys47.

Enfin, la présente invention concerne également les conjugués PEG-hirudine obtenus par le procédé décrit précédemment et leur emploi comme agent thérapeutique destiné à traiter les thromboses.

Les exemples ci-après, au regard des figures en annexe, sont destinés à illustrer la présente invention, pour

la préparation du PEG activé, celle d'un conjugué PEG-hirudine et l'activité anti-thrombotique du conjugué obtenu.

La figure 1 représente un chromatogramme obtenu après chromatographie du monométhylétherPEG 5.000-rHV2-Lys47 (MPEG 5.000-rHV2-Lys47) sur la colonne RP300. L'élution des protéines est suivie au cours du temps par une mesure de la densité optique (DO) à 215 nm.

La figure 2 représente un chromatogramme obtenu après chromatographie du PEG 50.000-rHV2-Lys47 sur la colonne Lichrosphère RP8. L'élution des protéines est suivie au cours du temps par une mesure de la DO à 280 nm.

La figure 3 représente, sous forme de graphe, la concentration plasmatique de rHV2-Lys47 libre ou conjuguée en fonction du temps, lors de l'expérience sur une courte période décrite à l'exemple 7c.

La figure 4 représente, sous forme de graphe, le pourcentage d'activité de rHV2-Lys47 libre ou conjuguée en fonction du temps, lors de l'expérience sur une longue période décrite à l'exemple 7c.

**EXEMPLE 1 : préparation du monométhyléther PEG (5.000) (MPEG 5.000) activé.**

Toute la réaction d'activation du MPEG 5.000 s'effectue en milieu anhydre. Il convient donc dans un premier temps d'éliminer toute trace d'eau pouvant être présente dans les constituants du milieu réactionnel. Les stocks de MPEG 5.000 (Merck) et de N,N'-Carbonyldiimidazole (CDI) (Aldrich) sont conservés dans un dessicateur contenant du gel de silice pour capter l'eau. Du tamis moléculaire de 5 A (Prolabo) est séché dans un four monté à 400°C, puis on laisse descendre la température à 50°C et on ajoute le tamis moléculaire à 500 ml de dioxane (SDS) pour capter l'eau présente dans le solvant.

25 g (environ 4 mmoles) de MPEG sont ajoutés à 8,1g (environ 50 mmoles) de CDI et le mélange est lyophilisé au moins 2h. La poudre séchée est alors reprise dans 50 ml de dioxane et la réaction d'activation du MPEG 5.000 par le CDI s'effectue pendant 2h à 37°C sous agitation. Les molécules de MPEG 5.000 n'ayant qu'un seul groupement hydroxy libre, elles n'ont qu'un seul site d'activation possible.

La récupération du MPEG 5.000 activé se fait par précipitation à l'éther. Lorsque la température du milieu de réaction est descendue à 20°C ou moins, on ajoute 2 volumes d'éther en agitant ce qui fait précipiter instantanément le MPEG 5.000, alors que le CDI en excès et l'imidazole libéré lors de la réaction restent en solution. La solution est ensuite filtrée sur un filtre Whatmann 44, le filtre est lavé avec de l'éther et le précipitat de MPEG 5.000 activé lyophilisé est repris dans 50 ml de dioxane et reprécipité par de l'éther comme décrit ci-dessus. Après filtration, le MPEG 5.000 activé est lyophilisé toute la nuit, puis il est conservé dans un dessicateur contenant du gel de silice de façon à le préserver de l'eau.

**EXEMPLE 2 : Préparation du PEG 50.000 activé.**

Toute la réaction d'activation du PEG 50.000 s'effectue en milieu anhydre. Pour éliminer toute trace d'eau, le PEG 50.000, le CDI et le dioxane sont traités comme décrit dans l'exemple 1.

19,2g (environ 384 µmoles) de PEG 50.000 sont ajoutés à 1g (environ 6 mmoles) de CDI et le mélange est lyophilisé au moins 2h. La poudre séchée est alors reprise dans 50 ml de dioxane et la réaction d'activation du PEG 50.000 par le CDI s'effectue pendant 15h à 60°C sous agitation. Les molécules de PEG 50.000 ont deux groupements hydroxy libres, il y a donc deux sites potentiels d'activation par molécules de PEG 50.000.

Le PEG 50.000 activé est récupéré par précipitation à l'éther comme décrit dans l'exemple 1. On récupère 18,6g de PEG 50.000 activé.

**EXEMPLE 3 : Couplage du MPEG (5.000) activé avec de l'hirudine rHV2-Lys47.**

**A. Conjugaison du MPEG 5.000 à rHV2-Lys47**

3g (environ 0.5 mmole) de MPEG 5.000 activé de l'exemple 1 sont mélangés à 15 mg (environ 2 µmoles) de rHV2-Lys 47 dans 25ml de tampon borate 100 mM à pH 8,4. Le milieu de réaction est laissé 12h à 4°C sous agitation. Le MPEG 5.000 activé se désactivant rapidement au contact avec l'eau, on rajoute 3g de MPEG 5.000 activé dans le milieu de réaction environ toutes les 12h, 4 fois de suite. Après la quatrième addition de MPEG 5.000 activé, le milieu de réaction est laissé encore 48h à 4°C.

**B. Purification du conjugué MPEG 5.000-rHV2-Lys 47**

La purification du conjugué MPEG 5.000-rHV2-Lys47 se fait en 2 étapes :

i) une chromatographie d'échange d'anions permet déliminer l'excès de MPEG 5.000 qui n'est par retenu alors que le MPEG 5.000-rHV2-Lys 47 et rHV2-Lys47 libre sont adsorbés sur la colonne.

ii) une chromatographie de phase inverse permet de séparer rHV2-Lys47 libre résiduelle du MPEG 5.000-rHV2-Lys47.

1. Chromatographie d'échange d'anions sur Q-Sépharose.

10 ml de phase sont coulés dans une colonne Biorad de 14 mm de diamètre, et équilibrés avec 50 ml de tampon d'équilibration : borate 10 mM à un débit de 1 ml/min. Toute la chromatographie s'effectue à un débit de 1 ml/min. Le milieu de réaction contenant du MPEG 5.000-rHV2-Lys47 obtenu en A. est dilué au 1/10 avec de l'eau de façon à avoir une solution à 10 mM borate. On obtient un échantillon d'environ 250 ml qui est déposé sur la colonne de Q-Sépharose. La colonne est lavée avec 50 ml de tampon d'équilibration, puis l'élution s'effectue avec 10 ml de tampon borate 10 mM pH 8,5, NaCl 1 M, et la colonne est rééquilibrée dans du tampon d'équilibration. On récupère un échantillon de 20 ml contenant rHV2-Lys47 libre et le MPEG 5.000-rHV2-Lys47, qui correspondent aux 10 ml de l'élution plus les 10 premiers ml de la rééquilibration de la colonne.

2. Chromatographie sur phase inverse RP300.

Toute la chromatographie se fait à un débit de 1 ml/min. On utilise une colonne Aquapore RP300 de diamètre 95 mm et de longueur 250 mm (Applied Biosystems) et une HPLC Hewlett Packard HP 1090.

Le pH de l'échantillon obtenu en 1, contenant rHV2-Lys47 libre et du MPEG 5.000-rHV2-Lys47, est abaissé à 2 avec du tampon d'équilibration TFA 10 mM dans l'eau, ce qui augmente le volume à 35 ml final. 5 ml d'échantillon sont injectés sur la colonne RP300, puis la colonne est lavée avec du tampon d'équilibration de façon à éliminer les sels présents dans l'échantillon. L'élution s'effectue alors par un gradient de 40 min, allant de 0 à 100% acétonitrile, contenant du TFA 10 mM, puis la colonne est lavée avec 10 à 20 ml de tampon d'équilibration. L'élution des protéines est suivie par une mesure de la DO à 215 nm. L'opération est effectuée 7 fois de façon à chromatographier les 35 ml de l'échantillon. Un chromatogramme est présenté à la figure 1. Deux fractions sont récupérées :

- la fraction 1 correspond au pic dont le temps de rétention est de 15 min. et contient rHV2-Lys47 libre.
- la fraction 2 correspond aux pics dont les temps de rétention sont compris entre 19 et 22 min., et contient le MPEG 5.000-rHV2--Lys47.

La comparaison des pics d'élution de rHV2-Lys47 (fraction 1) et du MPEG 5.000-rHV2-Lys47 indique que plus de 95% de rHV2-Lys47 est couplé au MPEG 5.000.

Les 7 fractions 1 contenant rHV2-Lys47 libre obtenus lors des 7 chromatographies sont mélangées, puis lyophilisées toute la nuit, et reprises dans du tampon PBS (voir tableau). On procède de la même façon pour les 7 fractions 2 contenant le MPEG 5.000-rHV2-Lys47.

La séquence N-terminale du conjugué obtenu a pu être déterminée normalement, ce qui indique que le MPEG 5.000 activé ne se fixe pas sur la fonction amine terminale de rHV2-Lys47.

**EXEMPLE 4 : Couplage du PEG 50.000 activé avec de l'hirudine rHV2-Lys47.**

**A. Conjugaison du PEG 50.000 à rHV2-Lys47.**

2g (environ 40 µmole) de PEG 50.000 activé de l'exemple 2 sont mélangés à 2 mg (environ 280 nmoles) de rHV2-Lys47 dans 5 ml de tampon borate 100mM à pH 8,4. Le milieu de réaction est laissé 24h à 4°C sous agitation. Le PEG 50.000 activé se désactivant rapidement au contact avec l'eau, 2g de PEG 50.000 activé contenus dans 5 ml de tampon borate 100mM sont rajoutés dans le milieu de réaction toutes les 24h, 4 fois de suite. On récupère 36 ml d'une solution de PEG 50.000-rHV2-Lys47.

**B. Purification du conjugué PEG 50 000-rHV2-Lys47**

Comme pour le MPEG 5.000-rHV2-Lys47, la purification du conjugué PEG 50.000-rHV2-Lys47 se fait en 2 étapes :

i) une chromatographie d'échange d'anions et,
ii) une chromatographie de phase inverse.

1. Chromatographie d'échange d'anions sur Q-Sépharose.

10 ml de phase sont coulés dans une colonne Biorad de 14 mm de diamètre, et équilibrés avec 50 ml de tampon d'équilibration : borate 10 mM à pH 8,5 à un débit de 1 ml/min. Toute la chromatographie s'effectue à un débit de 1 ml/min. Le milieu de réaction contenant du PEG 50.000-rHV2-Lys47 obtenu en B. est dilué au 1/10 avec de l'eau de façon à avoir une solution à 10mM borate. On obtient un échantillon d'environ 360 ml qui est chromatographié sur la colonne de Q-Sépharose en 3 fois. 120 ml d'échantillons sont déposés sur la colonne de Q-Sépharose qui est ensuite lavée avec 50 ml de tampon d'équilibration, puis l'élution s'effectue avec 10 ml de tampon borate 10 mM pH 8,5, NaCl 1 M, et la colonne est rééquilibrée dans du tampon d'équilibration. On récupère un échantillon de 40 ml contenant rHV2-Lys47 libre et le PEG 50.000-rHV2-Lys47, qui correspondent aux 10 ml de l'élution plus les 30 premiers ml de la rééquilibration de la colonne.

2. Chromatographie sur phase inverse Lichrosphère RP8

Toute la chromatographie se fait à un débit de 1 ml/min. On utilise une colonne Lichrosphère RP8 5µ (Hewlett Packard) de diamètre 4 mm et de longueur 125 mm et une HPLC Hewlett Packard HP 1090.

Le pH de l'échantillon obtenu en 1 contenant rHV2-Lys47 libre et du PEG 50.000-rHV2-Lys47 est abaissé à 1, 5 avec 200 µl de TFA pur. La colonne est équilibrée dans du tampon d'équilibration (TFA 10mM, eau). 20 ml d'échantillon sont injectés sur la colonne RP8, puis la colonne est lavée avec du tampon d'équilibration de façon à éliminer les sels présents dans l'échantillon. L'élution s'effectue alors par un gradient en 40 min, allant de 0 à 100% acétonitrile contenant du TFA 10mM, et la colonne est lavée avec 10 à 20 ml de tampon d'équilibration. L'élution des protéines est suivie par une mesure de la DO à 215 nm. L'opération est effectuée 2 fois de façon à chromatographier les 40 ml de l'échantillon. Un chromatogramme est représenté à la Figure 2. Deux fractions sont récupérées:

- la fraction 1 correspond au pic dont le temps de rétention est de 15 min. et contient rHV2-Lys47 libre.

- la fraction 2 correspond aux pics dont les temps de rétention sont compris entre 20 et 30 min. et contient le PEG 50.000-rHV2-Lys47.

La comparaison des pics d'élution de rHV2-Lys47 (fraction 1) et du PEG 50.000-rHV2-Lys47 (fraction 2) indique que plus de 70% de rHV2-Lys47 est couplé au PEG 50.000.

Les 7 fractions 1 contenant rHV2-Lys47 libre obtenus lors des 2 chromatographies sont mélangées, puis lyophilisées toute la nuit, et reprises dans du tampon PBS. On procède de la même façon pour les 2 fractions 2 contenant le PEG 50.000-rHV2-Lys47.

**EXEMPLE 5 : quantification et activité des conjugués obtenus**

La quantification des conjugués obtenus dans les exemples 3 et 4 a été effectuée par 3 méthodes différentes :

- la chromatographie analytique sur phase inverse (HPLC analytique),
- le test d'activité antithrombine, et
- l'analyse des acides aminés

a) Chromatographie analytique

On utilise une colonne Lichrosphère RP8 5µ (Hewlett Packard) de diamètre 4 mm et de longueur 125 mm et une HPLC Hewlett Packard HP 1090. Le débit est de 1 ml/min. pendant toute la chromatographie.

La colonne Lichrosphère RP8 est équilibrée dans un tampon d'équilibration acétonitrile 30%, TFA 10 mM, eau 70%. Environ 25 µg de PEG-rHV2-Lys47 contenus dans 500 µl de PBS sont injectés sur la colonne, et après lavage de la colonne avec le tampon d'équilibration, l'élution s'effectue par un gradient en 40 min allant de 30 à 65% acétonitrile dans de l'eau contenant du TFA 10mM, puis la colonne est rééquilibrée dans le tampon d'équilibration. L'élution des protéines est suivie par une mesure de la DO à 215 nm, caractéristique des protéines. La surface des pics d'élution présents sur le chromatogramme est proportionnelle à la quantité de protéiné injectée sur la colonne. Une chromatographie d'une quantité connue de rHV2-Lys47 dans les mêmes conditions que celles décrites ci-dessus, permet de corréler la surface du pic d'élution à la quantité de rHV2-Lys47 injectée.

b) activité anti-thrombotique

L'activité de l'hirudine est dosée par un test d'inhibition de la thrombine. Le substrat utilisé dans ce test est du Chromozyme PL (Tosylglycyl-prolyl-lysine-4-nitranilide acétate) qui est clivé par la thrombine et libère un chromophore qui absorbe la lumière à 410 nm.
La vitesse d'apparition du chromophore dépend de la quantité de thrombine active présente dans le milieu de réaction. En présence d'hirudine, la thrombine est inhibée et la vitesse d'apparition du chromophore est plus lente.

**Produits utilisés pour le test d'activité :**

- rHV2-Lys47 : 2,5 mg/ml conservé dans la glace
- Thrombine : 3,3 µg/ml (1U/ml), dans du tampon cinétique, conservée dans de la glace
- Chromozyme PL (Boehringer) : 6,66 mg/ml conservé à température ambiante
- Tampon cinétique :

  Tris HCl 50 mM
  NaCl 0,1 M
  0,1 % PEG 6000
  pH 7,9
  Thermostaté à 37°C.

La réaction est suivie à 410 nm dans un spectrophotomètre thermostaté à 37°C par un bain marie. Entre 0 et 100 µl d'hirudine sont mélangés, dans une cuve de spectromètre en plastique de 1ml, à 50µl de thrombine dans le tampon de cinétique de façon à avoir un volume final de 980 µl. Les cuves sont préincubées 3 min. à 37°C pour que l'hirudine puisse se fixer à la thrombine. 20 µl de chromozyme sont rajoutés dans chaque cuve et, après agitation la DO est lue à 410 nm toutes les minutes pendant 3 minutes. Le résultat est donné en DO/min.
L'activité spécifique de rHV2-Lys47 étant connue (15.000 ATU/mg), le dosage de l'activité du conjugué permet de le quantifier.

c) analyse des acides aminés

L'analyse d'acides aminés est effectuée de manière classique. Le conjugé est hydrolysé par des vapeurs d'HCl 6N, puis les acides aminés sont conjugués au Phénylisothiocyanate (PITC) dans du n-heptane à 5%, qui absorbe la lumière à 254 nm. Les conjugués d'acides aminés sont injectés sur une colonne de phase inverse C18, puis sont élués en utilisant un gradient permettant de séparer les différents conjugués. La conjugaison des acides aminés au PITC et la chromatographie se font sur un analyseur d'acides aminés Applied Biosystems 130A. Le temps de rétention sur la colonne de phase inverse, est spécifique de chaque conjugué d'acide aminé. Une solution standard de conjugués d'acides aminés permet de définir ces temps de rétention et de quantifier les acides aminés présents dans l'hydrolysat du conjugué de rHV2-Lys47. Connaissant la composition en acides aminés de rHV2-Lys47, on peut déterminer la quantité exacte de rHV2-Lys47 ainsi analysée.
Les résultats obtenus pour les conjugués des exemples 3 et 4 sont donnés dans le tableau I ci-dessous.

**TABLEAU I**

| Exemple | Volume ml | Quantification (en µg) | | |
|---|---|---|---|---|
| | | HPLC analytique | Test d'activité | Analyse d'acides aminés |
| 3 | 17 | 8200 | 8000 | 9194 |
| 4 | 1,7 | 1688 | 1070 | 1208,8 |

Le fait que pour ces deux exemples, les tests d'activité donnent des résultats de quantification, voisins de ceux obtenus par HPLC analytique ou analyse d'acides aminés montre clairement que près de 100% des conjugués PEG-hirudine obtenus sont actifs (entre 85 et 98% pour l'exemple 3 et entre 63 et 88% pour l'exemple 4).
Ces résultats permettent d'envisager leur emploi ultérieur comme agent anti-thrombotique sans nécessiter une purification supplémentaire des conjugués actifs.

**EXEMPLE 6 : Mesure de la constante d'inhibition de l'hirudine rHV2-Lys47 et de ses deux conjugués du MPEG 5.000 et du PEG 50.000.**

L'analyse de la constante d'inhibition est effectuée selon la méthode décrite par R.S. Stone et J. Hofsteenge dans Biochemistry, 1986, 25, pages 4628, et Biochemistry, 1991, 4, pages 295-300.

L'hirudine inhibe la thrombine selon un mécanisme de type compétitif. La constante d'inhibition peut donc être calculée comme suit :

$$Ki = \frac{Ki'}{1+S/K_M},$$

avec

Ki = constante d'inhibition,
Ki' = constante d'inhibition apparente,
$K_M$ = 3,63 µM = constante de Michaëlis, et
s = concentration de substrat.

Ki' et S peuvent être mesurés.

Les résultats obtenus sont donnés dans le Tableau II ci-dessous.

| TABLEAU II | | | |
|---|---|---|---|
| **Inhibiteur** | **Ki' (pM)** | | **Ki(pM)** |
| **rHV2-Lys47** | **29,30** | **1,77** | **1,03** |
| **MPEG 5000-rHV2-Lys47** | **28,78** | **1,43** | **1** |
| **PEG 50 000-rHV2-Lys47** | **34,12** | **1,04** | **1,19** |

La fixation de MPEG 5.000 ou de PEG 50.000 n'affecte pas la constante d'inhibition du complexe rHV2-Lys47-thrombine. Ces résultats montrent que les conjugués PEG-hirudine sont des inhibiteurs de la thrombine aussi efficaces que l'hirudine libre.

**EXEMPLE 7 : Tests de pharmacocinétique in vivo**

Pour évaluer la demi-vie de rHV2-Lys47 libre et du PEG-rHV2-Lys47 dans le plasma des rats, 2 types d'expériences ont été effectuées :

- expérience sur une courte période : elle ne dure que 5 à 6 h maximum pendant lesquelles les rats sont anesthésiés ;
- expérience sur une longue période : elle dure 48 h et les rats sont conscients pendant toute la durée de l'expérience.

Pour les expériences on utilise des rats de la souche Sprague Dawley de 300 g. 5 jours avant le début des expériences, les rats sont séparés dans des cages individuelles, ils sont acclimatés à un cycle jour-nuit de 13h, et ils ont de l'eau et de la nourriture à volonté.

Les échantillons de sang sont récoltés dans des tubes en plastique contenant une solution de citrate de sodium 3,2%, à un rapport de 1 volume pour 4 volumes de sang.

Le plasma est préparé à partir du sang dans les 10 min suivant la collecte par une centrifugation à 4.000 g pendant 5 min à température ambiante. Les échantillons de plasma sont stockés à -80°C. Le conjugué employé est le MPEG 5.000-rHV2-Lys47, pour l'expérience sur une courte période et le MPEG 5.000-rHV2-Lys47 ainsi que le PEG 50.000-rHV2-Lys47 pour l'expérience sur une longue période.

a) Expérience sur une courte période

Chaque expérience est effectuée en parallèle sur 2 rats. Le jour de l'expérience les rats sont anesthésiés par une administration de pentobarbital de sodium (50 mg/kg) par voie intrapéritonéale. Un cathéter est placé dans la veine jugulaire droite pour administrer 500 µg de rHV2-Lys47 sous forme libre (contrôle) ou conjuguée. Un second

cathéter est placé dans la carotide gauche pour collecter les échantillons de sang.

Des échantillons de sang sont collectés avant l'injection de rHV2-Lys47 ou de PEG-rHV2-Lys 47 ($t_o$), et à 5 min, 10 min, 20 min, 1h, 2h, 4h, 5h et 6h après l'injection.

b) Expérience sur une longue période

Chaque expérience est effectuée en parallèle sur 2 rats. Le jour de l'expérience 500 µg de rHV2-Lys47 sous forme libre (contrôle) ou conjuguée, sont administrés dans la veine de la queue des rats. Les échantillons de sang sont collectés sous une anesthésie douce à l'éther, à partir de la veine de la queue du rat, avant l'injection de rHV2-Lys47 ou de PEG-rHV2-Lys 47 ($t_o$), et à 5 min, 8h et 24h après l'injection. Excepté à ces moments, les rats sont conscients pendant toute la durée de l'expérience.

c) Analyse des échantillons de plasma

La quantité de rHV2-Lys47 sous forme libre (contrôle) ou conjuguée est déterminée par un test ELISA et par un test d'activité dans les essais sur une courte période. On effectue une gamme étalon avec une solution de rHV2-Lys47 ou de MPEG 5.000-HV2-Lys47 bien quantifiée, diluée dans du plasma récolté à $t_o$, de façon à être dans des conditions similaires à celles des échantillons prélevés. Les échantillons de plasma sont dilués dans du tampon cinétique pour que la concentration en rHV2-Lys47 soit comprise dans la gamme étalon. Dans les essais sur une longue période, la quantité de rHV2-Lys47 sous forme libre ou conjuguée (au MPEG 5.000 et PEG 50.000) est déterminée par un test d'activité antithrombine. Les résultats de ces analyses sont donnés sur les graphes représentés Figure 3 pour les essais sur une courte période, et Figure 4 pour les essais sur une longue période. Il existe une dégradation C-terminale de rHV2-Lys47 dans le plasma, ce qui explique la différence de résultats de quantification obtenus par le test ELISA et par le test d'activité. En effet, un des 2 anticorps utilisés pour le test ELISA reconnait la partie C-terminale de rHV2-Lys47, et ne reconnait donc plus les molécules dégradées restées actives.

Cependant on observe que, pour une même quantité de rHV2-Lys47 injectée aux rats sous forme libre ou sous forme de conjugué au MPEG 5.000, la concentration plasmatique de MPEG 5.000-rHV2-Lys47 est 7 à 8 fois supérieure à celle de rHV2-Lys47 libre. Pour une même efficacité d'inhibition, on peut donc injecter 7 à 8 fois moins de MPEG 5.000-rHV2-Lys47 que de rHV2-Lys47 libre.

Les résultats de pharmacocinétique présentés à la Figure 4, confirment que le temps de demi-vie du MPEG 5.000-rHV2-Lys47 est augmenté par rapport à celui de rHV2-Lys47. Par contre celui du PEG 50.000-rHV2-Lys47 est nettement supérieur aux deux précédents. En effet, 3 heures après l'injection, on mesure une activité antithrombine supérieure à 50% chez les rats traités avec le conjugué PEG 50.000-rHV2-Lys47 et d'environ 30% chez les rats traités avec le conjugué MPEG 5.000-rHV2-Lys47 par rapport à l'activité initialement mesurée avant injection. On mesure une activité antithrombine inférieure à 10%, 8 heures après l'injection de rats avec l'hirudine sous forme libre.

Le coût de production du MPEG 5.000-rHV2-Lys47 préparé selon l'invention n'étant que faiblement augmenté par rapport au coût de production de rHV2-Lys47 libre, l'utilisation du MPEG 5.000-rHV2-Lys47 en pharmacologie peut être d'un coût nettement diminué par rapport à l'utilisation de rHV2-Lys47.

## Revendications

1. Procédé de préparation d'un conjugué polyéthylèneglycol(ou dérivés)-peptide (PEG-peptide) comprenant les étapes d'activation du polyéthylèneglycol ou ses dérivés (PEG) dans un solvant anhydre approprié, de récupération du PEG activé et de couplage du PEG activé avec le peptide, **caractérisé en ce que** le PEG est activé à l'aide de l'agent d'activation carbonyldiimidazole, et la récupération du PEG activé est effectuée par précipitation à partir-du mélange d'activation à l'aide d'un solvant organique hydrophobe.

2. Procédé selon la revendication 1, **caractérisé en ce que** le solvant organique hydrophobe est choisi parmi les solvants aprotiques apolaires.

3. Procédé selon l'une des revendications 1 ou 2, **caractérisé en ce que** le solvant organique hydrophobe est choisi parmi les éthers aliphatiques et les hydrocarbures.

4. Procédé selon l'une des revendications 1 à 3, **caractérisé en ce que** le solvant organique hydrophobe est sélectionné parmi l'éther éthylique, le pentane, l'hexane ou l'heptane, de préférence l'éther éthylique.

5. Procédé selon l'une des revendications 1 à 4, **caractérisé en ce que** le précipité est ultérieurement filtré puis

lyophilisé.

6. Procédé selon l'une des revendications 1 à 5, **caractérisé en ce que** la réaction d'activation est effectuée dans un solvant anhydre approprié choisi parmi les solvants aprotiques polaires.

7. Procédé selon l'une des revendications 1 à 6, **caractérisé en ce que** le solvant anhydre est choisi parmi le dioxane, le diméthylformamide, l'acétonitrile, le diméthylsulfoxyde ou le tétrahydrofurane.

8. Procédé selon l'une des revendications 1 à 7, **caractérisé en ce que** le PEG a un poids moléculaire compris entre 2.000 et 100.000, avantageusement compris entre 2.000 et 50.000, et est choisi parmi le polyéthylèneglycol et les monoesters aliphatiques en $C_1$-$C_6$ du polyéthylèneglycol.

9. Procédé selon l'une des revendications 1 à 8, **caractérisé en ce que** l'agent d'activation est le carbonyldiimidazole.

10. Procédé selon la revendication 9, **caractérisé en ce que** le rapport molaire entre l'agent d'activation et le PEG est compris entre 5 et 50, de préférence compris entre 10 et 20.

11. Procédé selon l'une des revendication 1 à 10, **caractérisé en ce que** le peptide est de l'hirudine ou un de ses variants.

12. Procédé de préparation d'un conjugué polyéthylèneglycol(ou dérivés)-hirudine (PEG-hirudine) comprenant :

    a) l'activation du polyéthylèneglycol ou ses dérivés (PEG) par réaction avec le carbonyldiimidazole dans un solvant anhydre approprié,
    b) la récupération du PEG activé par précipitation à l'aide d'un solvant hydrophobe,
    c) la condensation du PEG activé sur l'hirudine ou ses variants en milieu faiblement alcalin, et
    d) la purification du conjugué obtenu.

13. Procédé selon la revendication 12, **caractérisé en ce que** la réaction d'activation est effectuée dans un solvant anhydre approprié choisi parmi les solvants aprotiques polaires.

14. Procédé selon l'une des revendications 12 ou 13, **caractérisé en ce que** le solvant anhydre approprié est sélectionné parmi le dioxane, le diméthylformamide, l'acétonitrile, le diméthylsulfoxyde ou le tétrahydrofurane.

15. Procédé selon l'une des revendications 12 à 14, **caractérisé en ce que** le solvant organique hydrophobe est choisi parmi les solvants aprotiques apolaires.

16. Procédé selon l'une des revendications 12 à 15, **caractérisé en ce que** le solvant organique hydrophobe est choisi parmi les éthers aliphatiques et les hydrocarbures.

17. Procédé selon l'une des revendications 12 à 16, **caractérisé en ce que** le solvant hydrophobe est choisi parmi l'éther éthylique, le pentane, l'hexane ou l'heptane, de préférence l'éther éthylique.

18. Procédé selon l'une des revendications 12 à 17, **caractérisé en ce que** le rapport molaire entre le carbonyldiimidazole et le PEG est compris entre 5 et 50, de préférence est compris entre 10 et 20.

19. Procédé selon l'une des revendications 12 à 18, **caractérisé en ce que** le conjugué obtenu est purifié par les deux étapes successives suivantes :

    - de chromatographie sur échange d'anions pour éliminer l'excès de PEG qui n'aurait pas réagit, et
    - de chromatographie de phase inverse pour séparer le conjugué obtenu de l'hirudine libre résiduelle.

20. Procédé selon l'une des revendications 12 à 19, **caractérisé en ce que** le PEG e un poids moléculaire compris entre 2.000 et 100.000, avantageusement compris entre 2000 et 50.000 et est choisi parmi le polyéthylèneglycol, ou les monoesters aliphatiques en $C_1$ - $C_6$ du polyéthylèneglycol.

21. Conjugué polyéthylèneglycol (ou dérivés)-hirudine (PEG-hirudine) dans lequel le polyéthylèneglycol ou ses dérivés (PEG) est lié à l'hirudine sur au moins une fonction amine du peptide, différente de la fonction amine terminale, à

l'exception de cette fonction amine terminale, ledit conjugué étant obtenu selon le procédé de l'une des revendications 12 à 20.

**22.** Conjugé selon la revendication 21 **caractérisé en ce que** le PEG a un poids moléculaire compris entre 2.000 et 100.000, avantageusement 2.000 et 50,000, et est choisi parmi le polyéthylèneglycol, ou les monoesters aliphatiques en $C_1$-$C_6$ du polyéthylèneglycol.

**23.** A titre de médicament, le conjugué selon l'une des revendications 21 et 22, pour le traitement des thromboses.

**Claims**

**1.** Process for preparing a polyethylene glycol (or derivatives)-peptide (PEG-peptide) conjugate comprising the steps of activating polyethylene glycol or its derivatives (PEG) in an appropriate anhydrous solvent, recovering the activated PEG and coupling the activated PEG with the peptide, **characterized in that** PEG is activated by means of the activating agent carbonyldiimidazole, and the recovery of the activated PEG is performed by precipitation from the activation mixture by means of a hydrophobic organic solvent.

**2.** Process according to Claim 1, **characterized in that** the hydrophobic organic solvent is chosen from apolar aprotic solvents.

**3.** Process according to one of Claims 1 or 2, **characterized in that** the hydrophobic organic solvent is chosen from aliphatic ethers and hydrocarbons.

**4.** Process according to one of Claims 1 to 3, **characterized in that** the hydrophobic organic solvent is selected from ethyl ether, pentane, hexane or heptane, preferably ethyl ether.

**5.** Process according to one of Claims 1 to 4, **characterized in that** the precipitate is subsequently filtered and then freeze-dried.

**6.** Process according to one of Claims 1 to 5, **characterized in that** the activation reaction is carried out in an appropriate anhydrous solvent chosen from polar aprotic solvents.

**7.** Process according to one of Claims 1 to 6, **characterized in that** the anhydrous solvent is chosen from dioxane, dimethylformamide, acetonitrile, dimethyl sulphoxide or tetrahydrofuran.

**8.** Process according to one of Claims 1 to 7, **characterized in that** the PEG has a molecular weight of between 2,000 and 100,000, advantageously of between 2,000 and 50,000, and is chosen from polyethylene glycol and $C_1$-$C_6$ aliphatic monoesters of polyethylene glycol.

**9.** Process according to one of Claims 1 to 8, **characterized in that** the activating agent is carbonyldiimidazole.

**10.** Process according to Claim 9, **characterized in that** the molar ratio between the activating agent and the PEG is between 5 and 50, preferably between 10 and 20.

**11.** Process according to one of Claims 1 to 10, **characterized in that** the peptide is hirudin or one of its variants.

**12.** Process for preparing a polyethylene glycol (or derivatives) -hirudin (PEG-hirudin) conjugate, comprising:

a) the activation of polyethylene glycol or its derivatives (PEG) by reaction with carbonyldiimidazole in an appropriate anhydrous solvent,
b) the recovery of the activated PEG by precipitation by means of a hydrophobic solvent,
c) the condensation of the activated PEG with hirudin or its variants, in a slightly alkaline medium, and
d) the purification of the conjugate obtained.

**13.** Process according to Claim 12, **characterized in that** the activation reaction is carried out in an appropriate anhydrous solvent chosen from polar aprotic solvents.

**14.** Process according to one of Claims 12 or 13, **characterized in that** the appropriate anhydrous solvent is chosen from dioxane, dimethylformamide, acetonitrile, dimethyl sulphoxide or tetrahydrofuran.

**15.** Process according to one of Claims 12 to 14, **characterized in that** the hydrophobic organic solvent is chosen from apolar aprotic solvents.

**16.** Process according to one of Claims 12 to 15, **characterized in that** the hydrophobic organic solvent is chosen from aliphatic ethers and hydrocarbons.

**17.** Process according to one of Claims 12 to 16, **characterized in that** the hydrophobic solvent is chosen from ethyl ether, pentane, hexane or heptane, preferably ethyl ether.

**18.** Process according to one of Claims 12 to 17, **characterized in that** the molar ratio between carbonyldiimidazole and PEG is between 5 and 50, preferably between 10 and 20.

**19.** Process according to one of Claims 12 to 18, **characterized in that** the conjugate obtained is purified by the following two successive steps:

- of anion-exchange chromatography to remove the excess PEG which may have not reacted, and
- of reversed-phase chromatography to separate the conjugate obtained from the residual free hirudin.

**20.** Process according to one of Claims 12 to 19, **characterized in that** the PEG has a molecular weight of between 2,000 and 100,000, advantageously of between 2,000 and 50,000, and is chosen from polyethylene glycol or $C_1$-$C_6$ aliphatic monoesters of polyethylene glycol.

**21.** Polyethylene glycol (or derivatives)-hirudin (PEG-hirudin) conjugate, in which polyethylene glycol or its derivatives (PEG) is bound to hirudin via at least one amine functional group of the peptide, different from the terminal amine functional group, with the exception of this terminal amine functional group, the said conjugate being obtained according to the process of one of Claims 12 to 20.

**22.** Conjugate according to Claim 21, **characterized in that** the PEG has a molecular weight of between 2,000 and 100,000, advantageously 2,000 and 50,000, and is chosen from polyethylene glycol or $C_1$-$C_6$ aliphatic monoesters of polyethylene glycol.

**23.** As medicinal product, the conjugate according to one of Claims 21 and 22, for the treatment of thromboses.

**Patentansprüche**

**1.** Verfahren zur Herstellung eines Konjugats aus Polyethylenglykol (oder Derivaten)-Peptid (PEG-Peptid), umfassend die Schritte Aktivierung des Polyethylenglykols oder seiner Derivate (PEG) in einem geeigneten wasserfreien Lösungsmittel, Isolieren des aktivierten PEG und Koppeln des aktivierten PEG mit dem Peptid, dadurch charakterisiert, daß das PEG mit Hilfe des Aktivierungsmittels Carbonyldiimidazol aktiviert wird, und daß die Isolierung des aktivierten PEG durch Ausfällung ausgehend von der Aktivierungsmischung mit Hilfe eines hydrophoben organischen Lösungsmittels erfolgt.

**2.** Verfahren nach Anspruch 1, dadurch charakterisiert, daß das hydrophobe organische Lösungsmittel ein aprotisches apolares Lösungsmittel ist.

**3.** Verfahren nach einem der Ansprüche 1 oder 2, dadurch charakterisiert, daß das hydrophobe organische Lösungsmittel ausgewählt wird aus den aliphatischen Ethern und den Kohlenwasserstoffen.

**4.** Verfahren nach einem der Ansprüche 1 bis 3, dadurch charakterisiert, daß das hydrophobe organische Lösungsmittel ausgewählt wird aus Ethylether, Pentan, Hexan oder Heptan, bevorzugt Ethylether.

**5.** Verfahren nach einem der Ansprüche 1 bis 4, dadurch charakterisiert, daß der Niederschlag schließlich filtriert und anschließend lyophilisiert wird.

**6.** Verfahren nach einem der Ansprüche 1 bis 5, dadurch charakterisiert, daß die Aktivierungsreaktion in einem geeigneten wasserfreien Lösungsmittel durchgeführt wird, das ausgewählt wird aus polaren aprotischen Lösungsmitteln.

**7.** Verfahren nach einem der Ansprüche 1 bis 6, dadurch charakterisiert, daß das wasserfreie Lösungsmittel ausgewählt wird aus Dioxan, Dimethylformamid, Acetonitril, Dimethylsulfoxid oder Tetrahydrofuran.

**8.** Verfahren nach einem der Ansprüche 1 bis 7, dadurch charakterisiert, daß das PEG ein Molekulargewicht zwischen 2000 und 100000, vorteilhafterweise zwischen 2000 und 50000 aufweist und ausgewählt wird aus Polyethylenglykol und den aliphatischen ($C_1$-$C_6$)Monoestern von Polyethylenglykol.

**9.** Verfahren nach den Ansprüchen 1 bis 8, dadurch charakterisiert, daß das Aktivierungsmittel Carbonyldiimidazol ist.

**10.** Verfahren nach Anspruch 9, dadurch charakterisiert, daß das Molverhältnis zwischen Aktivierungsmittel und PEG zwischen 5 und 50, bevorzugt zwischen 10 und 20 liegt.

**11.** Verfahren nach einem der Ansprüche 1 bis 10, dadurch charakterisiert, daß das Peptid Hirudin oder eine seiner Varianten ist.

**12.** Verfahren zur Herstellung eines Konjugats Polyethylenglykol (oder Derivaten)-Hirudin (PEG-Hirudin), umfassend:

a) Aktivierung von Polyethylenglykol oder seinen Derivaten (PEG) durch Reaktion mit Carbonyldiimidazol in einem geeigneten wasserfreien Lösungsmittel,
b) Isolierung des aktivierten PEG durch Ausfällung mit Hilfe eines hydrophoben Lösungsmittels,
c) Kondensation des aktivierten PEG mit Hirudin oder seinen Varianten in schwach alkalischem Medium, und
d) Reinigung des erhaltenen Konjugats.

**13.** Verfahren nach Anspruch 12, dadurch charakterisiert, daß die Aktivierungsreaktion in einem geeigneten wasserfreien Lösungsmittel durchgeführt wird, das ausgewählt wird aus den polaren aprotischen Lösungsmitteln.

**14.** Verfahren nach einem der Ansprüche 12 oder 13, dadurch charakterisiert, daß das geeignete wasserfreie Lösungsmittel ausgewählt wird aus Dioxan, Dimethylformamid, Acetonitril, Dimethylsulfoxid oder Tetrahydrofuran.

**15.** Verfahren nach einem der Ansprüche 12 bis 14, dadurch charakterisiert, daß das hydrophobe organische Lösungsmittel ausgewählt wird aus den apolaren aprotischen Lösungsmitteln.

**16.** Verfahren nach einem der Ansprüche 12 bis 15, dadurch charakterisiert, daß das hydrophobe organische Lösungsmittel ausgewählt wird aus den aliphatischen Ethern und den Kohlenwasserstoffen.

**17.** Verfahren nach einem der Ansprüche 12 bis 16, dadurch charakterisiert, daß das hydrophobe Lösungsmittel ausgewählt wird aus Ethylether, Pentan, Hexan oder Heptan, bevorzugt Ethylether.

**18.** Verfahren nach einem der Ansprüche 12 bis 17, dadurch charakterisiert, daß das Molverhältnis zwischen Carbonyldiimidazol und PEG zwischen 5 und 50, bevorzugt zwischen 10 und 20 liegt.

**19.** Verfahren nach einem der Ansprüche 12 bis 18, dadurch charakterisiert, daß das erhaltene Konjugat durch die folgenden beiden aufeinanderfolgenden Schritte gereinigt wird:

- Anionenaustausch-Chromatographie zur Entfernung von unreagiertem überschüssigem PEG, und
- Chromatographie mit umgekehrter Phase zur Abtrennung des erhaltenen Konjugats vom restlichen freien Hirudin.

**20.** Verfahren nach einem der Ansprüche 12 bis 19, dadurch charakterisiert, daß das PEG ein Molekulargewicht zwischen 2000 und 100000, vorteilhafterweise zwischen 2000 und 50000 aufweist und ausgewählt wird aus Polyethylenglykol oder den aliphatischen ($C_1$-$C_6$)Monoestern von Polyethylenglykol.

**21.** Konjugat aus Polyethylenglykol (oder Derivaten)-Hirudin (PEG-Hirudin), bei dem das Polyethylenglykol oder seine Derivate (PEG) an das Hirudin an mindestens einer Aminfunktion des Peptids gebunden ist, die von der terminalen

Aminfunktion verschieden ist, mit Ausnahme dieser terminalen Aminfunktion, wobei das Konjugat nach dem Verfahren eines der Ansprüche 12 bis 20 erhalten wurde.

**22.** Konjugat nach Anspruch 21, dadurch charakterisiert, daß das PEG ein Molekulargewicht zwischen 2000 und 100000, vorteilhafterweise zwischen 2000 und 50000 aufweist, und aus Polyethylenglykol oder den aliphatischen ($C_1$-$C_6$)Monoestern von Polyethylenglykol ausgewählt wird.

**23.** Konjugat nach einem der Ansprüche 21 und 22 als Medikament für die Behandlung von Thrombosen.

FIG_1

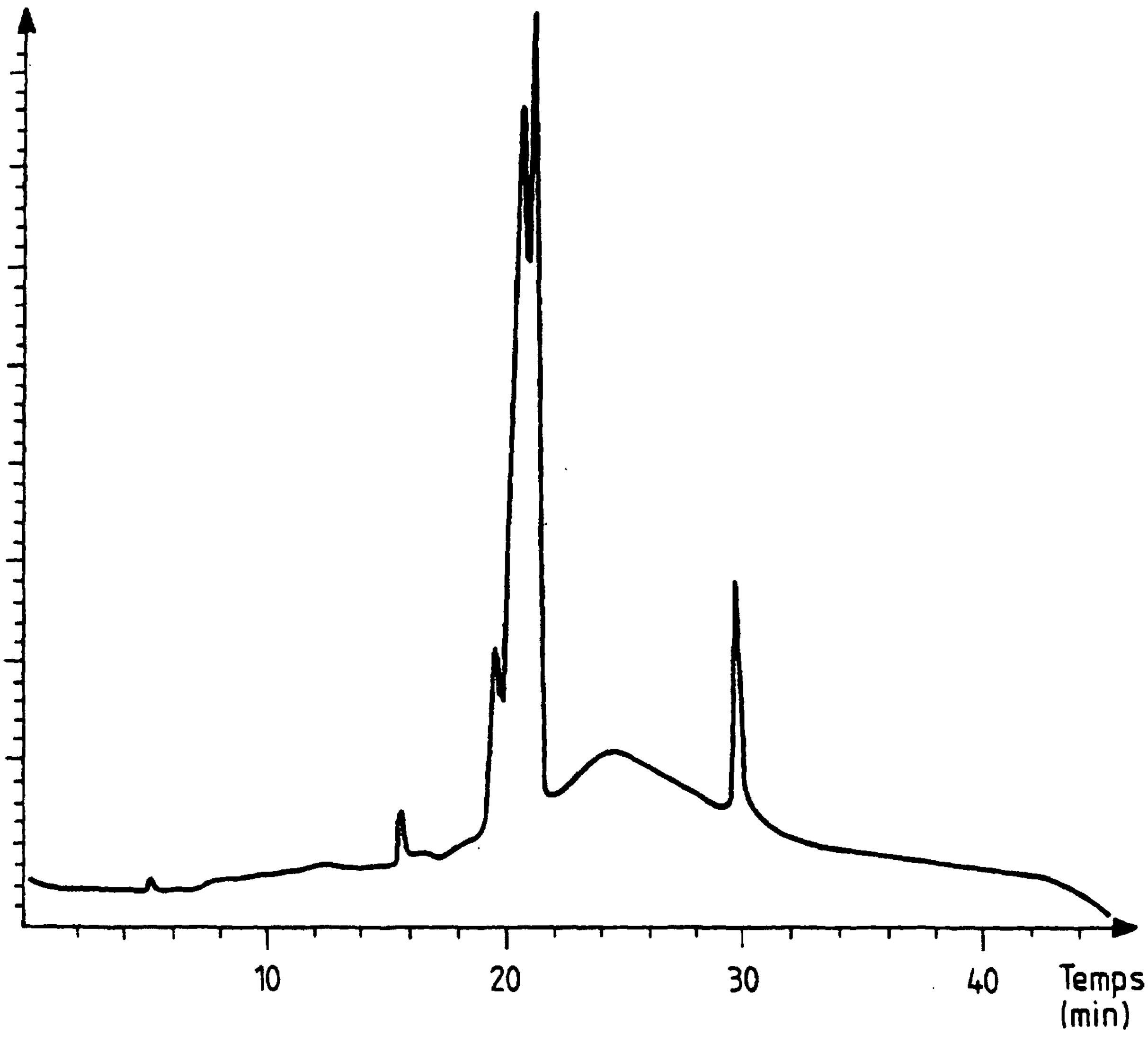

FIG_2

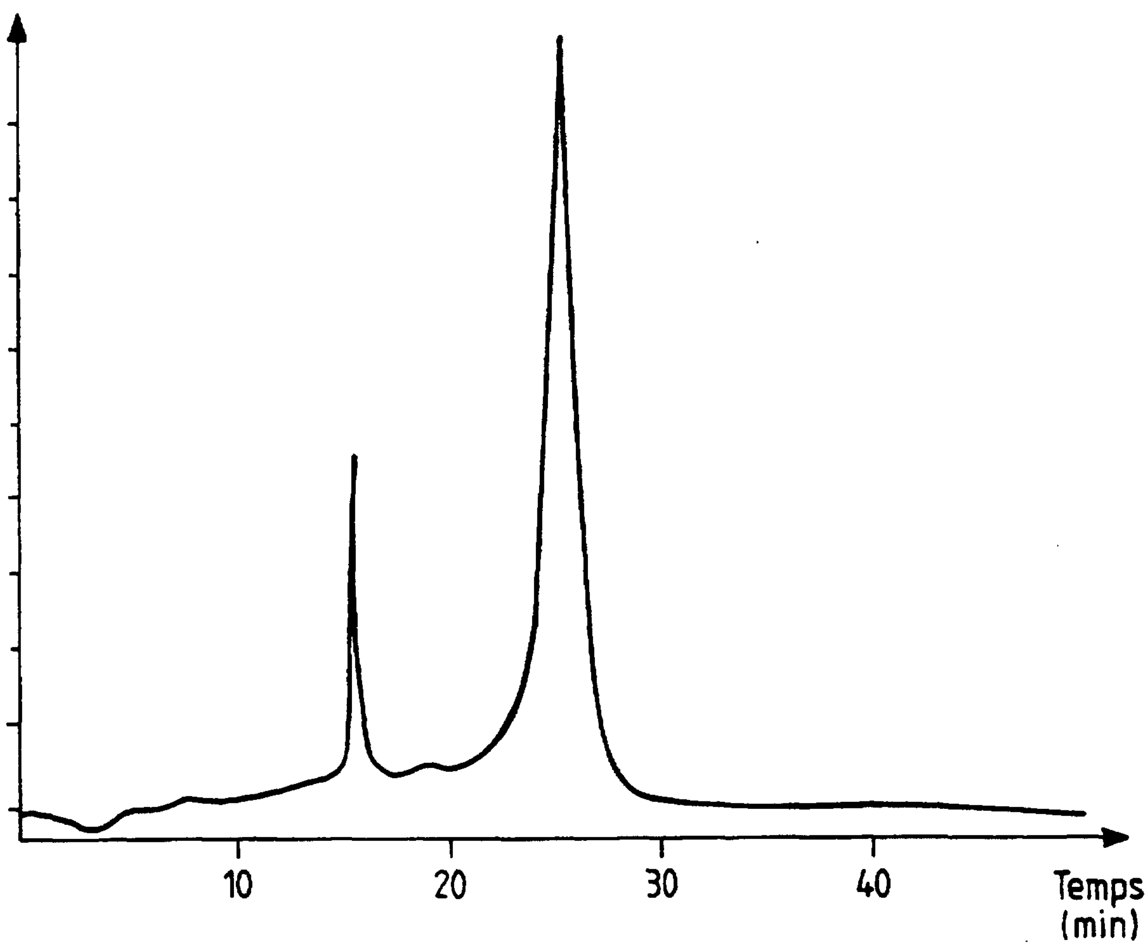

FIG_3

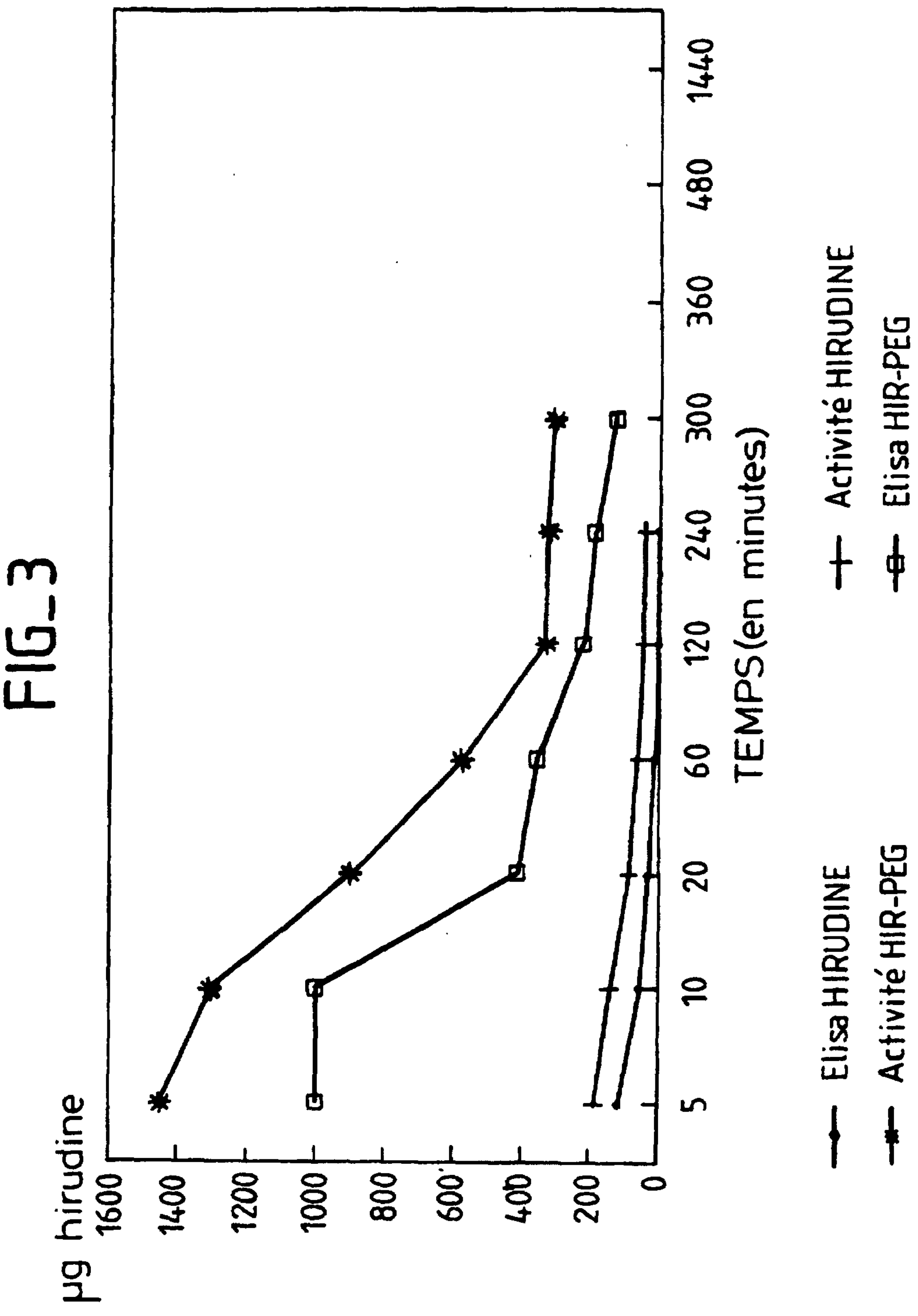
µg hirudine
1600
1400
1200
1000
800
600
400
200
0
5
10
20
60
120
240
300
360
480
1440
TEMPS(en minutes)
Elisa HIRUDINE
Activité HIR-PEG
Activité HIRUDINE
Elisa HIR-PEG

FIG_4

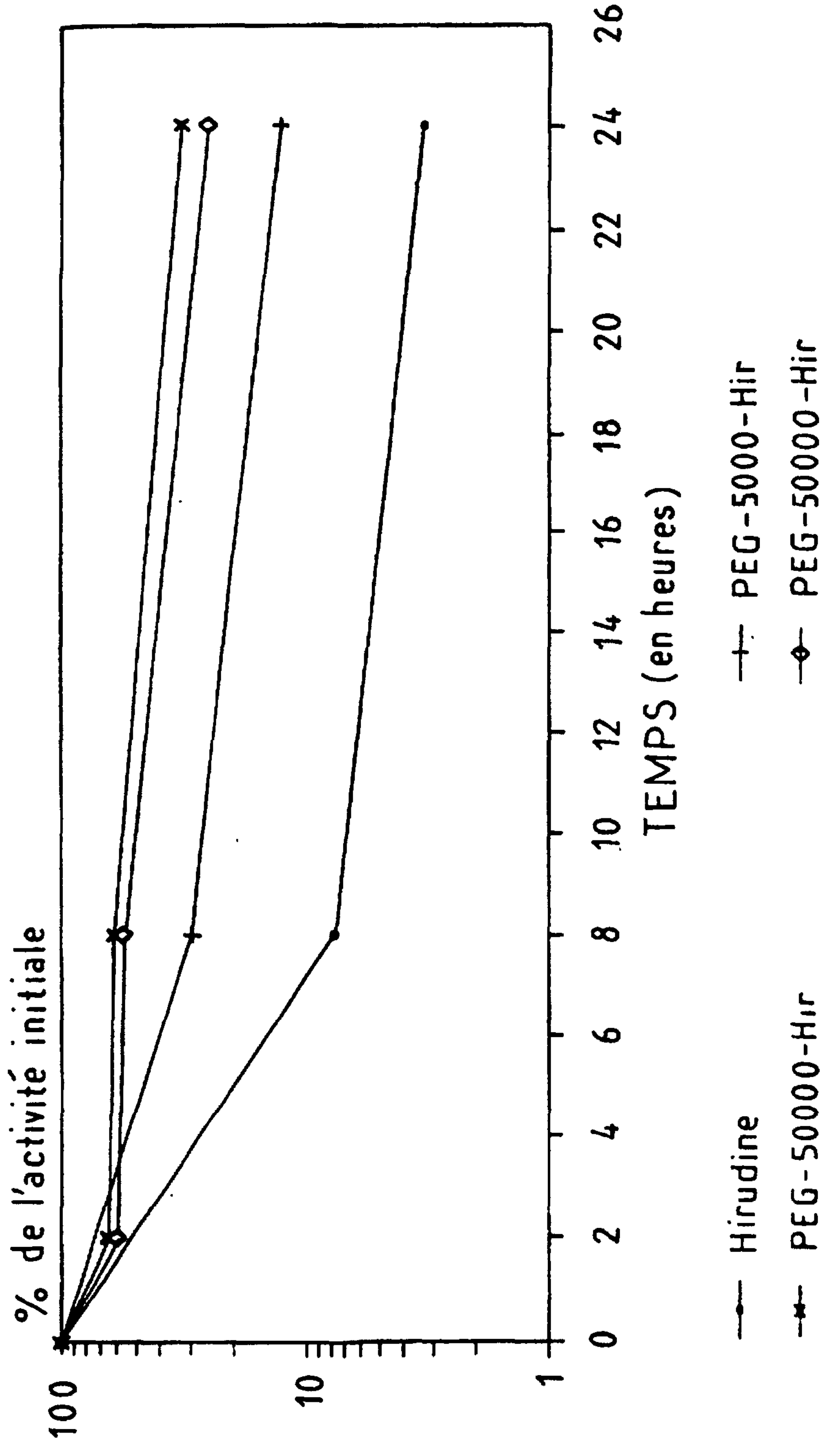
% de l'activité initiale
100
10
1
0
2
4
6
8
10
12
14
16
18
20
22
24
26
TEMPS (en heures)
Hirudine
PEG-50000-Hir
PEG-5000-Hir
PEG-50000-Hir